# EUROPÄISCHE PATENTSCHRIFT

(11) **EP 1 598 010 B1**
(45) Veröffentlichungstag und Bekanntmachung des Hinweises auf die Patenterteilung: **27.10.2010**
(21) Anmeldenummer: 05018332.6
(22) Anmeldetag: 30.10.2001
(51) Int. Cl.: A61B 5/15

(54) **System zur Blutentnahme**
System for withdrawing blood
Système de prélèvement de sang

(30) Priorität: 31.10.2000 DE 10053974
(43) Veröffentlichungstag der Anmeldung: 23.11.2005
(62) Teilanmeldung aus: 01982447.3
(73) Patentinhaber: Roche Diagnostics GmbH, 68305 Mannheim (DE); F.HOFFMANN-LA ROCHE AG, 4070 Basel (CH)
(72) Erfinder: Fritz, Michael, 68647 Biblis (DE); Sacherer, Klaus-Dieter, 87281 Kirchheim (DE); List, Hans, 64754 Hesseneck-Kailbach (DE); Weiss, Thomas, 68307 Mannheim (DE); Deck, Frank, 67150 Niederkirchen (DE); Immekus, Claudio, 79312 Emmendingen (DE)

(56) Entgegenhaltungen:
- EP-A- 0 565 970
- DE-A- 19 830 604
- DE-B- 2 803 345

## Beschreibung

Die vorliegende Erfindung betrifft ein System zur Entnahme von Körperflüssigkeit aus einem Körperteil, insbesondere der Fingerbeere, durch Erzeugung einer kleinen Stichwunde.

Im Bereich der klinischen Diagnostik ist es notwendig, Proben von Körperflüssigkeit, insbesondere Blutproben zu gewinnen, um darin Inhaltsstoffe nachweisen zu können. Wird eine größere Blutmenge benötigt, so wird diese im Regelfall mit einer Spritze oder dergleichen entnommen, wozu ein Blutgefäß gezielt angestochen wird. Die vorliegende Erfindung fällt jedoch in ein Gebiet, in dem lediglich Probenmengen im Bereich weniger µl oder darunter notwendig sind, um analytische Parameter zu bestimmen. Eine solche Vorgehensweise ist insbesondere zur Messung des Blutzuckerspiegels weit verbreitet, sie findet aber auch beispielsweise Anwendung um Gerinnungsparameter, Triglyzeride, HBA 1c oder Lactat zu bestimmen. Im Bereich der Diabeteserkrankung hat es sich durchgesetzt, daß Diabetiker selbst eine Überwachung des Blutzuckerspiegels vornehmen (sog. Home-Monitoring). Dies ist erforderlich, um durch gezielte Insulingaben einen Blutzuckerspiegel einzustellen, der sich im Normbereich befindet. Gerät ein Diabetiker hingegen in eine Unterzuckerung (Hypoglykämie) so kann dies eine Bewußtlosigkeit und sogar den Tod eines Patienten nach sich ziehen. Hat der Patient hingegen einen zu hohen Blutzuckerspiegel, so zieht dies gravierende Spätfolgen, wie Erblindungen und Gangräne nach sich. Für eine zur Messung des Blutzuckerspiegels erforderliche Blutentnahme haben sich kleine, handliche Blutentnahmegeräte, sog. Stechhilfen, eingebürgert, die vom Benutzer, Krankenhaus- und Pflegepersonal einfach und zuverlässig bedient werden können. Seit jüngerer Zeit sind auch Systeme zur Entnahme von interstitieller Flüssigkeit bekannt, mit der im Prinzip entsprechende Analysen durchgeführt werden können.

Ein sich in diesem Gebiet verstärkt abzeichnendes Problem besteht in der Kontamination und Verletzung durch benutzte Lanzetten. Bei vielen der im Handel befindlichen Geräten wird die Lanzette nach dem Stichvorgang entnommen oder ausgeworfen. Die in einem solchen Fall freiliegende Nadel der Lanzette kann zu Verletzungen führen, die unter Umständen Infektionen nach sich ziehen. In einigen Ländern gibt es daher bereits Bestrebungen, Blutentnahmesysteme, bei denen die Nadelspitze nach Benutzung frei zugänglich ist, zu verbieten.

In Dokumenten des Standes der Technik sind verschiedene Varianten von Blutentnahmesystemen beschrieben worden, bei denen die Nadel nach dem Stichvorgang geschützt ist. In dem Dokument US 5,314,442 wird eine Kappe beschrieben, in der eine Lanzette angeordnet ist. Zur Durchführung eines Stichvorganges wird die Lanzette durch einen Stößel oder dgl. so innerhalb der Kappe verschoben, daß die Nadel über eine Öffnung nach außen tritt. Nach dem Stich wird die Lanzette wieder ins Innere der Kappe zurückgezogen und flexible Elemente an der Lanzette sorgen dafür, daß die Lanzettennadel ohne Einwirkung des Stößels nicht mehr nach außen dringen kann. Vom Prinzip her ähnliche Systeme sind in den US Patenten 4,990,154 und 5,074,872, sowie der internationalen Anmeldung WO 00/02482 beschrieben. Ein weiteres System, bei dem das Zurückziehen einer Lanzette in eine Kappe durch eine eingebaute Feder erfolgt, ist in dem Dokument DE 198 55 465 beschrieben. Während die genannten Dokumente bereits das Problem einer Kontamination oder Verletzung des Benutzers lösen, so wird eine Ankopplung des Antriebsmechanismus an die Lanzette lediglich durch einen Preßsitz erzielt. Hierbei wird die Einstechtiefe der Nadel über einen Anschlag begrenzt. Es hat sich jedoch herausgestellt, daß das Auftreffen der Lanzette auf den Anschlag zu Vibrationen der Nadel führt, die den Schmerz beim Einstich erhöhen. Näheres zu dieser Problematik ist in EP 0 565 970 beschrieben.

Die Aufgabe der vorliegenden Erfindung bestand darin, ein Verfahren zur Entnahme von Körperflüssigkeit vorzuschlagen, das einerseits eine Kontamination oder Infektion durch benutzte Lanzetten vermeidet und andererseits dem Benutzer ein sehr schmerzarmes Einstechen ermöglicht. Eine weitere Aufgabe bestand darin, die Systeme des Standes der Technik zu vereinfachen, kostengünstiger zu machen und insbesondere ein Konzept vorzuschlagen, das verkleinert werden kann. Letzterer Punkt ist insbesondere wichtig, um ein System zur Verfügung zu stellen, das mit magazinierten Lanzetten arbeitet und dem Benutzer einen Wechsel auf eine noch unbenutzte Lanzette ermöglicht, ohne daß er hier zu komplizierte Handhabungsschritte vornehmen muß.

Die genannten Aufgaben werden durch Ausführungsformen für Systeme zur Entnahme von Körperflüssigkeit gelöst, die eine Antriebseinheit mit einem Stößel besitzen, der zur Ausführung eines Stechvorganges aus einer Ruheposition in eine Stechposition bewegt wird. Weiterhin beinhalten die Systeme eine Stecheinheit, in der sich eine Lanzette mit einer Nadel befindet, die in der Ruheposition des Stößels innerhalb der Stecheinheit angeordnet ist und die durch den Stößel bei Bewegung in die Stechposition so verschoben wird, daß die Nadel zumindest teilweise durch eine Austrittsöffnung aus der Stecheinheit austritt. Ein wesentliches Merkmal des Systems besteht darin, daß Stößel und Lanzette zur Durchführung des Stechvorganges durch Formschluß miteinander gekoppelt sind.

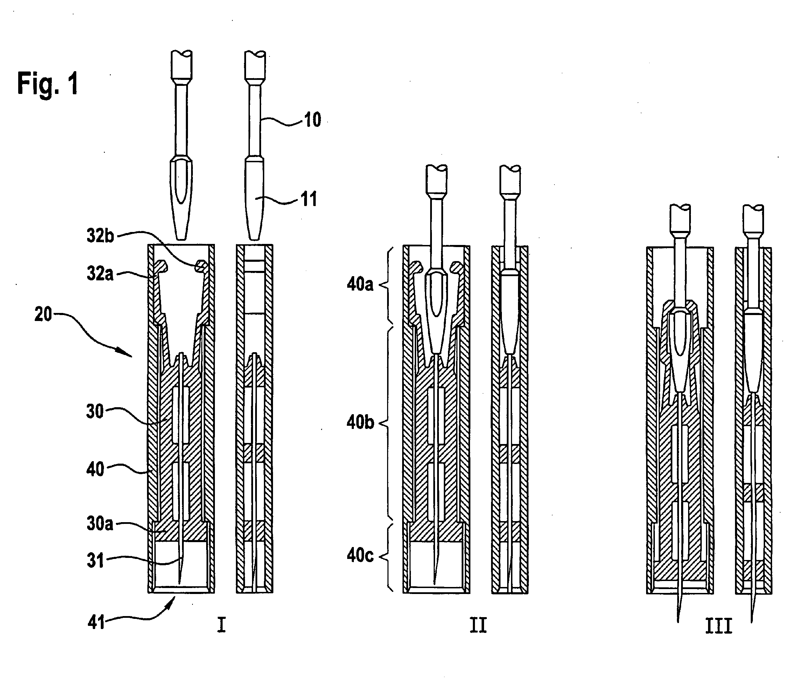

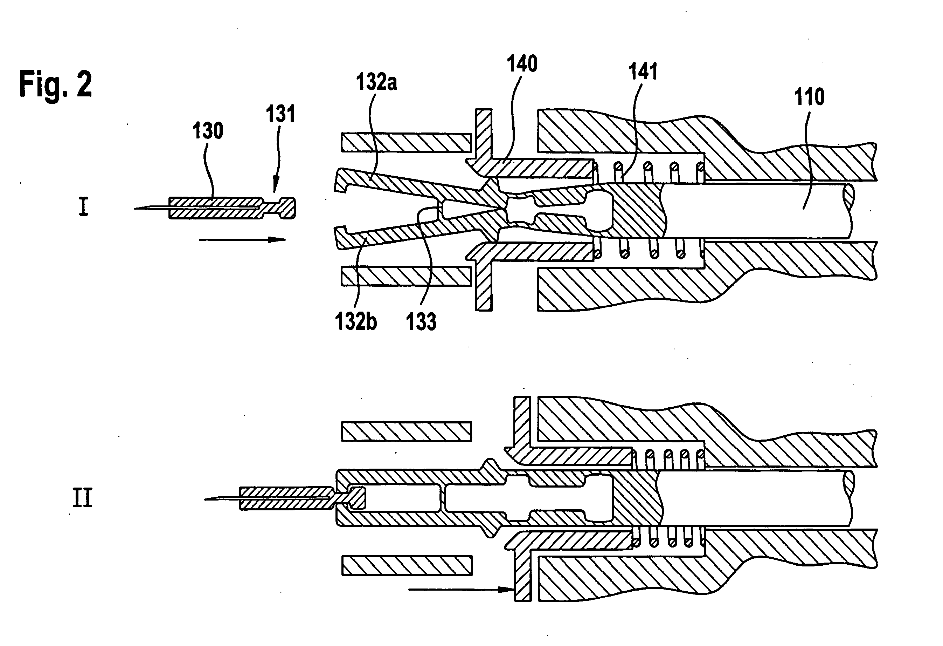

Für einen Formschluss im Sinne dieser Erfindung ist charakteristisch, dass durch ihn eine Kopplung von Lanzette und Antriebsstößel mittels eines geringen Kraftaufwandes möglich ist. Es gibt zwei prinzipielle Varianten einen Formschluss zu erzielen. Bei der ersten Variante schließt sich zur Kopplung von Lanzette und Antriebsstößel eine Form in der Weise, daß ein Halteelement umschlossen wird. Die sich schließende Form wird im Rahmen der Erfindung als Haltevorrichtung bezeichnet. Befindet sich die Haltevorrichtung an der Lanzette, so weist der Antriebsstößel ein Halteelement auf, befindet sich hingegen die Haltevorrichtung am Antriebsstößel, so ist das Halteelement an der Lanzette angeordnet. Die Figuren 1 und 2 zeigen praktische Ausgestaltungen. Vorzugsweise wird diese Variante des Formschlusses dadurch erzielt, dass durch eine Längsbewegung in Stichrichtung eine Querbewegung von Halteelementen einer (zunächst geöffneten) Haltevorrichtung erzielt wird, die sich hierbei um einen Haltebereich schließen. Vorzugsweise wid beim Schließen der Haltevorrichtung das Halteelement (zumindest teilweise) hintergriffen, so daß die Lanzette beim Zurückziehen des Antriebsstößels - zumindest bis zum etwaigen Öffnen der Haltevorrichtung - mitgenommen wird. Weiterhin ist es bevorzugt wenn Haltevorrichtung und Haltebereich geometrisch so aufeinander abgestimmt sind, daß nach erfolgtem Formschluß in Richtung der Stichbewegung keine oder nur eine geringe Toleranz besteht und die Bewegung des Antriebsstößels sowohl in Stichrichtung als auch in entgegengesetzter Richtung spielfrei in eine Bewegung der Lanzette umgesetzt wird. Dies kann erreicht werden, indem die Längsausdehnung der Kammer in der geschlossenen Haltevrrichtung mit der Längsausdehnung des Haltebereiches übereinstimmt oder nur unwesentlich größer ist (siehe Figur 1). Bei einer anderen Ausführungsform dieser Variante sind eine Ausnehmung im Haltebereich und Halteelemente an der Haltevorrichtung in ihrer Längsausdehnung so aufeinander abgestimmt, daß ein Transport in Stichrichtung im wesentlichen spielfrei möglich ist (siehe Figur 2).

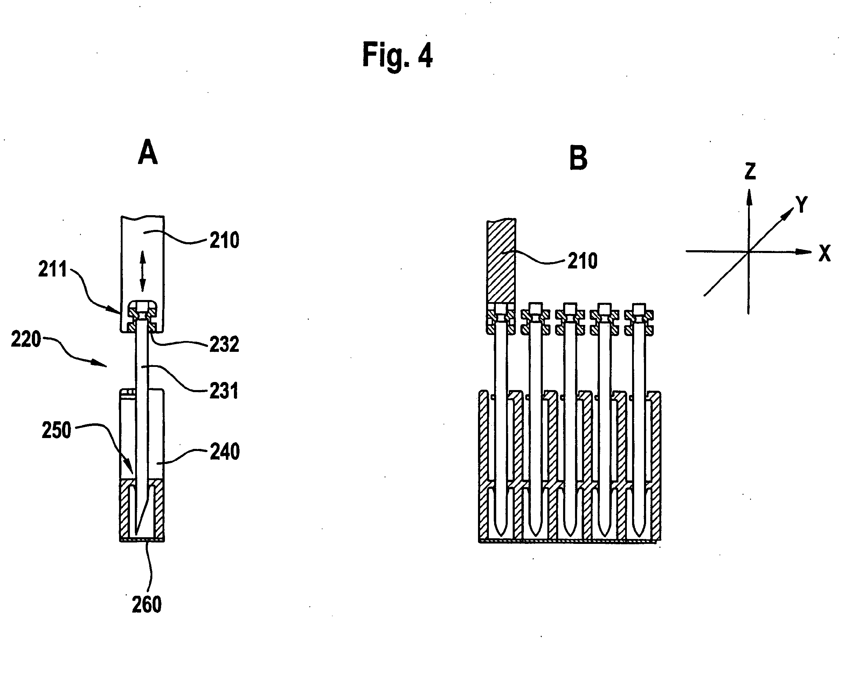

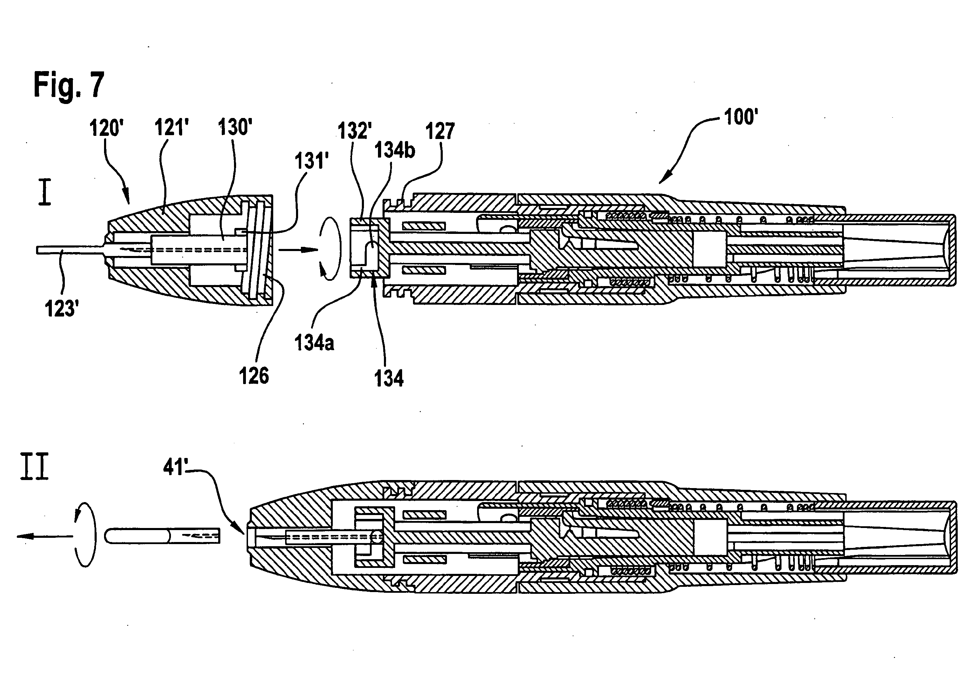

Bei einer zweiten Variate des Formschlusses sind sowohl Haltevorrichtung als auch Haltebereich im wesentlichen formstabil und ein Umschließen des Haltebereiches durch die Haltevorrichtung erfolgt durch Ineinanderbewegen der durch diese beiden Einheiten gegebenen Formen. Da die Einheiten formstabil sind, ist kein vollständiges Umschließen möglich, sondern die Formen müssen in soweit offen sein, daß sie ineinanderbewegt werden können. Das Ineinanderbewegen erfolgt dabei (zumindest mit einer Wegkomponente) quer zur Stichrichtung und es entsteht eine Kopplung, die ebenfalls im wesentlichen spielfrei in Stichrichtung ist (siehe Figur 4). Es ist schließlich auch eine Ankopplung durch eine Bewegung möglich, die sowohl Komponenten quer als auch parallel zur Stichrichtung aufweist (siehe Figur 7).

Das System zur Blutentnahme besitzt eine Antriebseinheit mit einem Stößel, durch den eine Lanzette aus einer Ruheposition in eine Stechposition bewegt wird. Im Stand der Technik sind eine Reihe von Antriebsmechaniken bekannt, die im Gebiet der Blutentnahmegeräte eingesetzt werden können. Insbesondere werden in großem Umfang Antriebsmechaniken eingesetzt, die ihre Energie aus einer zuvor gespannten Feder beziehen. Im Rahmen der vorliegenden Erfindung werden vorzugsweise Antriebseinheiten eingesetzt, die eine geführte Bewegung des Stößels und der Lanzette, bedingt durch die formschlüssige Kopplung, ermöglichen. Mit einer geführten Bewegung ist gemeint, daß die Lanzette sowohl über einen vorbestimmten Weg in den Körper gestochen als auch über einen vorbestimmten Weg-Zeitverlauf aus dem Körper herausgezogen wird. Bei den herkömmlichen, auf einer Kombination von einer Feder und einem Anschlag basierenden System des Standes der Technik wird der Weg-Zeitverlauf durch eine Vielzahl von Parametern, wie Herstellungstoleranzen (Reibungsverhältnisse in dem System, Stärke der Feder).als auch der Hautoberfläche beeinflußt. Es hat sich gezeigt, daß eine geführte Bewegung der Lanzette, wie sie beispielsweise über eine Führungskulisse wie in der EP 565 970 beschrieben, vorteilhaft bezüglich des Einstichschmerzes ist. Bezüglich der Antriebseinheit wird hiermit auf die bevorzugten Antriebmechaniken der EP 565970 und der US 4,924,879 Bezug genommen.

Einen wichtigen Aspekt stellt eine von der Antriebseinheit abnehmbare Stecheinheit dar, in der sich mindestens eine Lanzette befindet. Die Stecheinheit umfaßt ein Gehäuse, in dem die Lanzette in der Ruheposition angeordnet ist. Hierdurch kann vermieden werden, daß durch die Lanzette vor oder nach Benutzung Verletzungen hervorgerufen werden bzw. daß Kontaminationen erfolgen. Das Gehäuse kann so ausgestaltet sein, daß darin eine einzelne Lanzette angeordnet ist oder das Gehäuse kann die Form eines Magazines mit mehreren Lanzetten besitzen. Im Regelfall werden sich die Lanzetten innerhalb eines Magazines in voneinander separierten Kammern befinden, um eine Kontamination ungebrauchter Lanzetten durch bereits verwendete zu verhindern. Das Gehäuse der Stecheinheit ist vorteilhaft so ausgestaltet, daß es an der Antriebseinheit angebracht werden kann. Hierzu kann die Stecheinheit beispielsweise die Form einer Kappe aufweisen, die auf die Antriebseinheit aufgesteckt wird. Derartige Ausführungsformen sind beispielsweise in den Dokumenten US 5,314,442, US 4,990,154 und US 5,074,872 beschrieben. Es sind auch Ausführungsformen möglich, bei denen die Stecheinheit fest mit der Antriebseinheit verbunden ist, beziehungsweise einen integralen Bestandteil der Antriebseinheit bildet. Dies kann insbesondere bei Systemen mit Magazin vorteilhaft sein, so daß die gesamte Einheit nach Verbrauch des Magazins weggeworfen wird. Im Falle einer Stecheinheit in Form eines Magazines kann dieses beispielsweise nebeneinander angeordnete Kammern aufweisen, in denen sich Lanzetten befinden und die Kammern nacheinander relativ zur Antriebseinheit positioniert werden, so daß die Lanzetten an den Stößel der Antriebseinheit angekoppelt werden können. Besonders vorteilhaft ist auch ein Magazin in Form einer Trommel mit parallel zur Längsachse der Trommel angeordneten Kammern, in denen sich Lanzetten befinden. Ähnlich einer Revolvertrommel kann ein solches Magazin repetierbar an der Antriebseinheit angebracht sein.

Eine weitere Anforderung an die Stecheinheit liegt in der Sterilität der Lanzetten, die über einen langen Zeitraum gewährleistet sein muß. Eine Sterilität der Stecheinheit kann, wie im Stand der Technik üblich, durch Gammabestrahlung erzielt werden. Zum Aufrechterhalten der Sterilbedingungen kann die Stecheinheit in eine Umverpackung, beispielsweise einen Polyäthylenbeutel eingeschweißt werden. In einer anderen Ausführungsform werden die Öffnungen der Stecheinheit (zum Eintritt des Stößels und für den Austritt der Nadelspitze) durch Siegelfolien verschlossen. Dies können beispielsweise abziehbare Siegelfolien sein, die der Benutzer vor Benutzung entfernt. Vorteilhaft können jedoch auch dünne Folien verwendet werden, die bei der Benutzung von dem Stößel bzw. von der Nadelspitze durchstoßen werden, so daß dem Benutzer zusätzliche Handhabungsschritte erspart bleiben. Solche Folien können bereits integral im Herstellungsprozeß der Stecheinheit, der in der Regel ein Spritzgußprozeß ist, erzeugt werden.

Bei einer weiteren, vorteilhaften Ausführungsform ist die Nadelspitze durch ein Elastomer vor Kontamination geschützt, welches vor dem Stich abgezogen wird oder während des Stichvorganges durchstochen wird, um die Nadelspitze freizugeben. Ein derartiger Schutz der Nadel vor Kontamination ist in der Anmeldung WO 01/66010 beschrieben auf welche hiermit Bezug genommen wird.

Innerhalb der Stecheinheit befinden sich ein oder mehrere Lanzetten mit einer Nadel. Abgesehen von etwaigen Vorrichtungen an der Lanzette, die eine formschlüssige Ankopplung an einen Stößel ermöglichen, können im Rahmen dieser Erfindung Lanzetten eingesetzt werden, wie sie im Stand der Technik hinlänglich bekannt sind. Im Regelfall besitzt eine solche Lanzette einen Grundkörper aus Kunststoff, in dem eine Metallnadel angeordnet ist. Es sind jedoch auch Lanzetten ohne einen separaten Gründkörper möglich (beispielsweise Metallnadel mit einer Verdickung an hinteren Ende als Haltebereich).

Im Rahmen der Erfindung ist es von Bedeutung, daß der Stößel der Antriebseinheit und die Lanzette zur Ausführung des Stechvorganges durch Formschluß miteinander gekoppelt werden. Hierin unterscheidet sich die Erfindung wesentlich von dem Stand der Technik, wo eine mechanische Kopplung zwischen Lanzette und Antrieb über einen Preßsitz (US 5,314,442, US 4,990,154, US 5,074,872), eine Verrastung (WO 00/02482), eine Verklemmung (US 3,030,959) oder über einen einfachen Andruck (DE 198 55465) erfolgt. Ein Formschluß ist dadurch gekennzeichnet, daß eine mechanisch zuverlässige Kopplung zwischen dem Antrieb und der Lanzette erfolgt, ohne daß hierfür eine wesentliche Andruckkraft an die Lanzette in Richtung der Stechbewegung erfolgt. Bei den Vorrichtungen des Standes der Technik, die mit einem Preßsitz arbeiten, muß in der Kappe, die die Lanzette beinhaltet, ein Federelement (z. B. DE 198 55 465) oder ein Rückhalteelement (z. B. WO 00/02482) vorgesehen werden, das so ausgelegt ist, daß die Lanzette beim Ankoppeln der Stecheinheit an die Antriebseinheit nicht aus der Kappe austritt. Federelemente in der Stecheinheit führen jedoch zu erhöhten Herstellungskosten, was besonders gravierend ist, da es sich bei der Stecheinheit um Verbrauchsmaterial handelt. Zur Überwindung eines Rückhalteelementes ist hingegen eine zusätzliche Kraft seitens der Antriebseinheit notwendig und ein Überwinden führt auch zu Vibrationen der Nadel, die sich negativ auf den Stichschmerz auswirken. Im übrigen ist bei Systemen, die mit einem Preßsitz arbeiten, eine geführte Bewegung, die ein Zurückziehen der Lanzette beinhaltet problematisch, da hierdurch der Preßsitz gelöst werden kann. Die in der WO 00/02482 beschriebene Verrasterung löst dieses Problem zwar, sie ist jedoch technisch schwer zu realisieren. Insbesondere ist es schwierig, eine solche Verrasterung in einem kontinuierlichen Herstellungsprozeß zu etablieren, da schon geringfügige Schwankungen des Materials oder der Prozeßbedingungen eine Funktionsunfähigkeit der Vorrichtung nach sich ziehen. Ein weiterer Nachteil der in der WO 00/02482 beschriebenen Vorrichtung besteht darin, daß die Verrasterung in einem Wegebereich erfolgt, der zum Einstich in den Körper dient. Die bei der Verrasterung auftretenden Kräfteschwankungen und Vibrationen wirken sich nachteilig auf den Einstichschmerz aus. Nachteilig ist bei der Vorrichtung weiterhin, daß die Nadel nach dem Einstich im Körper verbleibt und nicht aktiv zurückgezogen wird. Ein Zurückziehen der Nadel erfolgt erst beim Abnehmen der Kappe von der Antriebsmechanik. Bei einem Formschluß von Antriebsstößel und Lanzette gemäß der vorliegenden Erfindung kann hingegen eine Verbindung zwischen Stößel und Lanzette erreicht werden, ohne daß hierzu eine besondere Kraft in Richtung des Stiches aufgewendet werden müßte und darüber hinaus kann die formschlüssige Verbindung vorteilhaft dazu genutzt werden, die Nadel nach dem Einstich aktiv zurückzuziehen. Durch diese Möglichkeit zur aktiven Steuerung der Weg-Zeitkurve der Nadel über die Antriebseinheit ist es möglich, den Stechvorgang sehr schmerzarm zu gestalten.

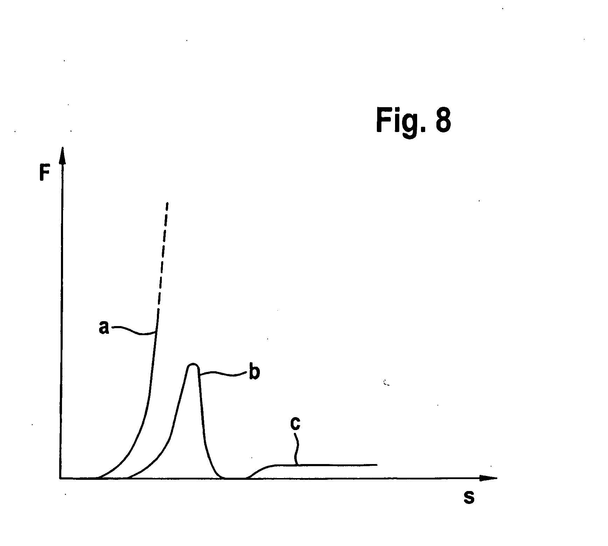

In Figur 8 sind schematische Kraft (F)-Weg (s)-Verläufe für Ankopplungsprozesse des Antriebs an die Lanzette für Preßsitz (a), Verrasterung (b) und Formschluß (c) dargestellt. Es ist zu erkennen, daß bei Verwendung des Preßsitzes die Kraft stark ansteigt, bis sich die Lanzette aus der Position, in der sie durch ein Halteelement oder eine Feder gehalten ist, löst. Bei einer Verrasterung steigt die Kraft beim Verrasten an und sinkt nach erfolgtem Schluß wieder. Bei einem Formschluß sind lediglich sehr geringe Kräfte zum Zusammenfahren der Halteelemente notwendig.

Eine weitere Eigenschaft des Formschlußes wird durch Vergleich mit dem Dokument US 3,030,959 deutlich. Bei einer Apparatur gemäß diesem US Patent werden Nadeln, die in einer Röhre angeordnet sind, nacheinander durch eine Klemmvorrichtung gehaltert, die einem Druckbleistift ähnlich ist. Neben den Kontaminationsproblemen durch gebrauchte Nadeln, die diese Apparatur ungelöst läßt, ist erkennbar, daß die Positionierung der Nadeln in axialer Richtung (d.h. in Stichrichtung) nicht definiert ist. So wie bei einem Druckbleistift die Länge der hervortretenden Bleistiftspitze vom Benutzer frei gewählt werden kann, hängt auch hier die axiale Positionierung der Nadel von der Einstellung durch den Benutzer ab. Bei einem Formschlußprinzip hingegen besitzen Lanzette und Antriebseinheit aufeinander abgestimmte Haltebereiche und Haltevorrichtungen. Durch die geometrische Ausgestaltung von Haltebereich und Haltevorrichtung kann erreicht werden, daß die axiale Positionierung der Lanzette wohl definiert ist und somit die Einstechtiefe genau kontrolliert werden kann. Durch die Verwendung eines Formschlußes kann somit sowohl eine Kraftspitze in axialer Richtung bei Kopplung von Lanzette und Antriebsstößel vermieden werden als auch eine exakte axiale Positionierung erzielt werden. Bei einem Formschluß schließt sich eine Form (Haltevorrichtung) um eine andere Form (Haltebereich). Unter einem Sich-schließen ist in diesem Sinne sowohl eine Bewegung von Vorrichtungsteilen quer zur Stichrichtung als auch alternativ ein formschlüssiges Ineinandergreifen zweier in ihrer Form unveränderter Formkörper gemeint. Bei einer bevorzugten Ausgestaltung ist die Haltevorrichtung geöffnet und schließt sich um den Haltebereich wenn dieser in die Haltevorrichtung eingeführt wird. Insbesondere kann dieses Schließen durch eine Längsbewegung erfolgen bei der die Längsbewegung in eine Bewegung von Haltelementen der Haltevorrichtung mit Komponenten quer zur Längsbewegungsrichtung umgewandelt wird. Eine praktisch vorteilhafte Möglichkeit, diese Umwandlung einer Längebewegung in eine Querbewegung zu erzielen besteht darin, die Haltevorrichtung mit der Längsbewegung in einen sich verjüngenden Kanal (z.B. in einer Hülse) einzuführen bzw. in diesem zu verschieben (siehe Figur 1). Unter sich verjüngend werden hierbei nicht nur sich kontinuierlich verjüngende Kanäle verstanden sondern allgemein Kanäle deren lichte Weite sich in der Längsrichtung verringert. Ein Kanal braucht in diesem Zusammenhang kein über eine Umfangsfläche geschlossener Körper zu sein. Wenn die Haltevorrichtung beispielsweise wie in Figur 1 zwei gegenüberliegende Haken aufweist, genügen zwei Wandungen deren innerer Abstand sich zueinander verringert.
Es besteht andererseits auch die Möglichkeit, die Haltevorrichtung durch Aufheben eines gespannten Zustandes schließen zu lassen (siehe Figur 2), was beispielsweise durch Bewegung der Haltevorrichtung in einem sich erweiternden Kanal möglich ist.

Die vorliegende Erfindung wird anhand von Figuren näher erläutert:
Figur 1: Querschnittsdarstellung durch eine Stecheinheit mit einer Haltevorrichtung an der Lanzette
Figur 2: Querschnittsdarstellung durch einen Systemausschnitt mit einer Haltevorrichtung an der Antriebseinheit
Figur 3: Querschnittsdarstellung durch ein Gesamtsystem und Schritte der Benutzung
Figur 4: Systemausschnitt mit formstabiler Haltevorrichtung an der Antriebseinheit
Figur 5: Magazin von Stecheinheiten
Figur 6: System aus Antriebseinheit und Stecheinheit
Figur 7: System mit einer formstabilen Haltevorrichtung
Figur 8: Kraft-Weg-Diagramm für verschiedene Ankopplungsarten
Figur 9: Stecheinheit mit Sterilschutz
Figur 10: Trommelförmiges Magazin

Figur 1 zeigt ein Blutentnahmesystem gemäß einer ersten Ausführungsform der Erfindung in den Betätigungsphasen I, II und III. In der Figur 1 sind lediglich Teilaspekte des Systems dargestellt. In der Darstellung fehlt die Antriebseinheit für den Stößel (10) sowie das Gehäuse der Antriebseinheit, an der die Stecheinheit (20) befestigt wird. Als Antriebseinheit für den Stößel (10) ist die in der EP 0 565 970 beschriebene Antriebsvorrichtung besonders geeignet.

Die in der Figur 1 dargestellten Phasen zeigen das Zustandekommen der formschlüssigen Kopplung zwischen dem Antriebsstößel (10) und der Lanzette (30), sowie den eigentlichen Stichvorgang. Für jede der drei Phasen sind jeweils 2 Querschnittsdarstellungen entlang der Längsachse des Systems in zueinander senkrechten Ebenen dargestellt. Aus der linken Darstellung der Phase I ist zu erkennen, daß die Lanzette (30) innerhalb einer Hülse (40) angeordnet ist. Die dargestellte Lanzette (30) besitzt einen Grundkörper, der aus Kunststoff gefertigt ist sowie eine darin eingespritzte Nadel (31) aus Stahl. An der der Nadelspitze abgewandten Seite weist die Lanzette eine Haltevorrichtung mit Halteelementen in Form zweier Haken (32a, 32b) auf. Beim Einfahren des Stößels (10) in die Lanzette gelangt ein verdickter Bereich am vorderen Ende des Stößels, der als Haltebereich (11) dient, zwischen die Haken (32a, 32b) und trifft schließlich auf das hintere Nadelende (Phase II). Es ist auch möglich, den Stößel statt auf das hintere Nadelende auf den Grundkörper der Lanzette auftreffen zu lassen. Ein direkter Kontakt mit der Nadel ist jedoch von Vorteil, da die Nadellänge produktionstechnisch sehr exakt kontrolliert werden kann und somit eine genaue Kontrolle der Einstechtiefe möglich ist. Beim weiteren Vordringen des Stößels schiebt er die Lanzette innerhalb der Hülse (40) in Richtung der Austrittsöffnung (41), so daß schließlich die Nadelspitze über die Austrittsöffnung übersteht und in ein darunter liegendes Gewebe einsticht. Aus dem Übergang von Phase 11 zur Phase III ist zu erkennen, daß sich die Haltevorrichtungen (32a, 32b) an der Lanzette um den Haltebereich (11) des Stößels schließen, sobald die Lanzette innerhalb der Hülse (40) verschoben wird. Die Haltevorrichtung an der Lanzette umgreift das Halteelement des Stößels so, daß eine formschlüssige Verbindung entsteht, mit der nicht nur eine Vorwärtsbewegung der Lanzette zur Ausführung eines Stiches sondern auch ein aktives, von der Antriebseinheit gesteuertes Zurückziehen der Lanzette im wesentlichen spielfrei möglich ist. Dies wird einerseits dadurch gewährleistet, daß das Ende des Haltebereiches auf dem Nadelende aufsitzt als auch durch die Haken (32a, 32b) welche das hintere Ende des Haltebereiches hintergreifen. Die Länge des Haltebereiches und die Längsausdehnung der Kammer in der geschlossenen Haltevorrichtung sind so aufeinander abgestimmt, daß ein spielfreier Antrieb der Lanzette in Stichrichtung gegeben ist. Durch die Anordnung der Lanzette bzw. der Haltevorrichtung in einer sich verjüngenden Hülse wird eine Umwandlung einer Längsbewegung der Lanzette in Stichrichtung in eine Querbewegung der Elemente der Haltevorrichtung erzielt, die einen Formschluß mit dem Haltebereich des Antriebs ermöglicht. Wie aus den Figuren zu erkennen ist, besitzt die Hülse (40) einen mittleren Bereich (40b), der gegenüber dem oberen Bereich (40a) verjüngt ist. Durch diese Verjüngung werden die Haken (32a, 32b) der Lanzette beim Verschieben der Lanzette in der Hülse in Richtung auf die Längsachse zusammengedrückt, so daß ein Umschließen des Haltebereiches (11) erfolgt. Als besonders vorteilhaft hat es sich erwiesen, die Stecheinheit (20) so auszugestalten, daß die Lanzette innerhalb der Hülse gehaltert wird, wenn keine Einwirkung des Stößels vorliegt. Hierdurch kann sichergestellt werden, daß sich die Nadel im unbetätigten Zustand innerhalb der Hülse (40) befindet und somit keine Verletzungen oder Kontamination durch eine herausragende Nadelspitze verursacht wird. Ein Hindurchrutschen der Lanzette durch die Hülse in Richtung der Austrittsöffnung (41) wird wirksam dadurch verhindert, daß die Haken (32a, 32b) einen Absatz aufweisen, der auf einer Kante des mittleren Bereiches (40b) aufliegt. Die Neigung dieser Kanten und die Flexibilität der Haken können so aneinander angepaßt werden, daß ein Einschieben in die Verjüngung einerseits mit geringer Kraft erfolgen kann und andererseits ein ungewolltes Hindurchrutschen effizient vermieden wird. Um ein Herausrutschen der Lanzette aus der Hülse, in der der Stichrichtung entgegengesetzten Richtung zu vermeiden, ist bei der dargestellten Ausführungsform ein verbreiterter Teil (40c) am unteren Ende der Hülse sowie ein damit korrespondierender verbreiterter Teil (30a) am unteren Ende des Lanzettengrundkörpers vorgesehen.

Wie aus Figur III hervorgeht, befindet sich die Nadelspitze beim Auftreffen des Haltebereiches (11) auf das Nadelende noch innerhalb der Hülse (40). Dies ist vorteilhaft, da die durch das Auftreffen erzeugte Erschütterung keinen Einfluß auf den Stichvorgang im Gewebe hat, wodurch Einstichschmerz durch eine solche Erschütterung vermieden wird.

Erfindungsgemäß ist es bevorzugt, wenn das System so ausgelegt ist, daß nach Durchlaufen der Phasen I, II und III eine Rückbewegung des Stößels (10) in umgekehrter Richtung erfolgt, so daß der Stößel von der Lanzette entkoppelt wird und sich die Lanzette wieder vollständig innerhalb der Hülse befindet. Für die Ankopplung des Stößels an die Lanzette gibt es zwei Hauptvarianten. Bei der ersten Variante sind Gehäuse, Antriebseinheit und Stecheinheit so aneinander angepaßt, daß sich der Stößel im Ausgangszustand vollständig außerhalb der Stecheinheit (20) befindet (wie für Phase I dargestellt). Ein Nachteil dieser Ausführungsform ist es, daß vom Stößel zum Durchführen eines Stiches ein verhältnismäßig langer Weg zurückgelegt werden muß. Ein Vorteil ist es jedoch, daß sich der Stößel vollständig außerhalb der Hülse befindet, so daß eine Querbewegung möglich ist. Dementsprechend kann die erste Variante vorteilhaft für Systeme mit einem Lanzettenmagazin eingesetzt werden, bei dem nacheinander verschiedene Hülsen unter den Stößel bewegt werden. Bei einer zweiten Variante wird durch Ankoppeln der Stecheinheit an die Antriebseinheit bereits eine Positionierung gemäß Phase II bzw. noch darüber hinaus in Richtung auf Phase III erreicht. Bei einer solchen Ausführungsform kann der Weg, den der Stößel vornehmen muß, sehr klein gehalten werden, was konstruktionstechnisch günstig ist.

Figur 2 zeigt eine zweite Ausführungsform der Erfindung, bei der die Lanzette (130) einen Haltebereich (131) aufweist und die Antriebseinheit eine Haltevorrichtung (132a, 132b) besitzt. In der Figur 2 ist wiederum der Bereich des Systems gezeigt, der zur Halterung der Lanzette dient, nicht jedoch die Antriebseinheit. Auch im Zusammenhang mit dieser Ausführungsform ist es vorteilhaft, eine Antriebseinheit einzusetzen, die den Stößel (110) geführt bewegt. Der Stößel trägt an seinem vorderen Ende eine Haltevorrichtung mit Halteelementen in Form zweier Haken (132a, 132b), die über eine flexible Brücke (133) (oder ein Gelenk) miteinander verbunden sind. Die Anordnung bildet ein Federelement. In der Phase I sind diese Haken gespreizt, da ihre hinteren Enden durch eine Hülse (140) zusammengehalten werden. Beim Einschieben der Lanzette (130) wird gleichzeitig die Hülse gegen eine Feder (141) verschoben, so daß die hinteren Enden der Haken freigegeben werden und sich die vorderen Enden der Haken formschlüssig um den Haltebereich (131) der Lanzette schließen. Der Haltebereich der Lanzette weist eine Ausnehmung (Vertiefungen) auf, in die die Haken der Haltevorrichtung eingreifen. Die Längsausdehnung (in Stichrichtung) der eingreifenden Enden der Haltevorrichtung und die Längsausdehnung der Ausnehmungen sind im wesentlichen gleich, so daß mit dieser Anordnung eine geführte, im wesentlichen spielfreie Stechbewegung ausgeführt werden kann.
Auch bei dieser Ausführungsform erfolgt eine Umwandlung einer Längsbewegung der Haltevorrichtung in eine Querbewegung der Halteelemente.

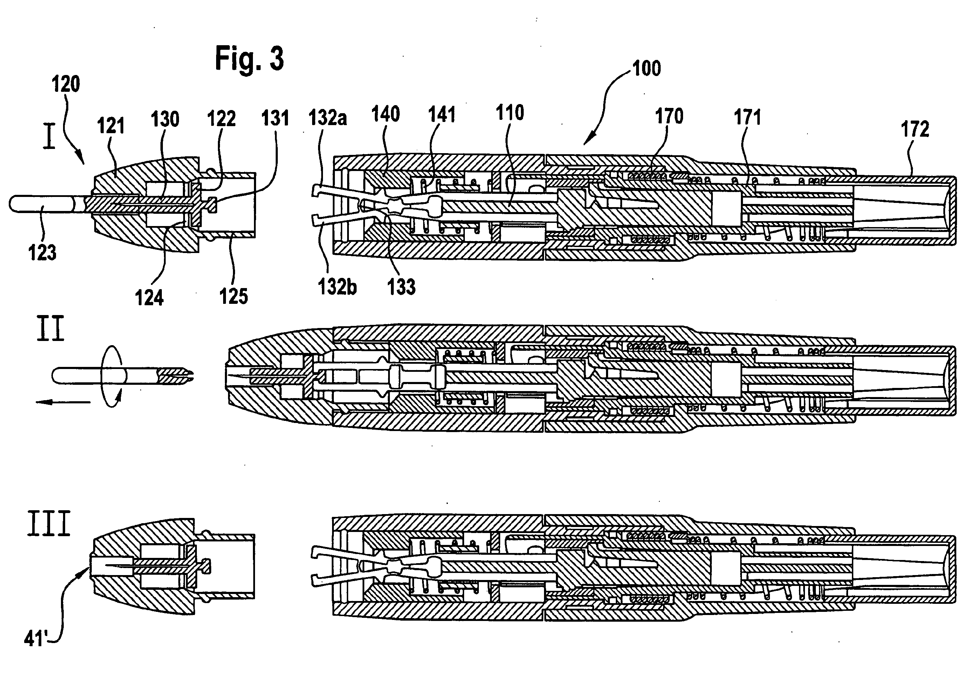

Figur 3 zeigt ein erfindungsgemäßes System, basierend auf dem formschlüssigen Ankopplungsprinzip gemäß Figur 2. Die Antriebseinheit 100 basiert auf dem Gerät Softclix®, welches in der EP B 0 565 970 beschrieben ist. Aus diesem Dokument geht insbesondere hervor, wie die durch die Antriebsfeder 170 vermittelte Drehbewegung der Hülse 171 in eine Translationsbewegung des Stößels 110 umgesetzt wird. Das Spannen der Antriebsfeder durch Niederdrücken des Druckknopfes 172 und eine hierfür geeignete mechanische Übersetzung sind in der europäischen Patentanmeldung mit dem Aktenzeichen EP 0 O10 2503.0 beschrieben. Die Antriebseinheit weist an ihrem vorderen Ende eine Haltevorrichtung mit zwei Halteelementen, im konkreten Fall Haken (132a, 132b) auf. Wie bereits zu Figur 2 ausgeführt, sind diese Haken in einem mittleren Bereich über eine flexible Brücke (133) bzw. ein Gelenk, miteinander verbunden. Auf der den Haken relativ zur Brücke (133) abgewandten Seite wird die Haltevorrichtung über eine Hülse (140) so gehaltert, daß die Haken geöffnet sind. Die Hülse (140) wird über eine in der Antriebsvorrichtung befindliche Feder (141) in Position gehalten. Aus Figur 3 I ist ferner eine Stecheinheit zu erkennen, bei der eine Lanzette (130) in einer Kappe (121) angeordnet ist. Die Lanzette besitzt an ihrem hinteren Ende einen Haltebereich (131), der von der Haltevorrichtung (132a, 132b) ergriffen wird. Der Außenkörper der Lanzette weist einen vorderen, schmalen Bereich auf sowie einen Flansch (122) zwischen diesem schmalen Bereich und dem Haltebereich (131). Die Spitze der Lanzette ist durch einen abdrehbaren Kunststoffkörper (123) vor Kontamination und mechanischen Einwirkungen geschützt. Die Kappe (121) weist in ihrem Inneren einen Durchgang für den schmaleren Bereich der Lanzette sowie einen im Querschnitt vergrößerten Bereich auf, der zur Aufnahme des Flansches (122) geeignet ist. Innerhalb dieses verbreiterten Kanales der Kappe ist ein Wulst (124) angeordnet, der ein selbständiges Hineinrutschen des Flansches in den erweiterten Kanal verhindert. Die Kappe besitzt weiterhin eine Hülse (125), welche dazu dient, die Hülse (140) der Antriebseinheit beim Aufsetzen der Stecheinheit auf die Antriebseinheit zurückzuschieben. Dieser Vorgang läßt sich durch Zusammenschau der Figuren 3I und 3II erkennen. Durch das Wegschieben der Hülse (140) mittels der Hülse (125) wird die Haltevorrichtung freigegeben, so daß sie den Haltebereich der Lanzette umfaßt, wie aus Figur 3II zu erkennen ist. Durch Drücken der Taste (172) und Abdrehen des Schutzteiles (123) von der Lanzette ist das System nunmehr für den Einsatz präpariert. Mit der in Figur 3II dargestellten Vorrichtung wird nunmehr ein Stechvorgang durchgeführt, in dem das vordere Ende der Kappe (120) auf ein Gewebeteil aufgesetzt und durch Betätigen eines Auslösemechanismus die Antriebseinheit aktiviert wird. Nach erfolgtem Stechvorgang wird die Kappe von der Antriebseinheit in Richtung der Längsachse abgezogen, wobei der Flansch (122) hinter den Wulst (124) zurückgezogen wird, so daß die kontaminierte Nadelspitze nicht mehr aus der Kappe austreten kann. Die Stecheinheit im Zustand gemäß Figur 3 III kann verworfen, oder nach Ankopplung an die Antriebseinheit für weitere Entnahmevorgänge verwendet werden.

Figur 4 zeigt eine dritte Ausführungsform der Erfindung, bei der der Formschluß zwischen Lanzette und Antrieb durch ein formschlüssiges Verbinden von geometrisch aneinander angepaßten Haltebereichen und Haltevorrichtungen erzielt wird. In der Figur 4A ist eine Stecheinheit (220) dargestellt, die eine Hülse (240) aufweist, in der sich eine Metallnadel (231) befindet. Die Hülse (240) besitzt eine dünne Querwand(250), die die Metallnadel relativ zur Hülse haltert. Diese Querwand wird vorzugsweise gleich beim Umspritzen der Nadeln mit Kunststoff geformt. Aufgrund der relativ geringen Dicke dieser Wand kann die mechanische Verbindung von Hülse und Nadel beim Stichvorgang gelöst werden, so daß die Nadel an der Wand (250) vorbeigleitet. An der Austrittsöffnung der Hülse ist diese mit einer dünnen Folie (260) verschlossen, die beim Stichvorgang durchstochen wird. An ihrem oberen Ende trägt die Nadel (231) einen angespritzten Haltebereich (232). Zur mechanischen Stabilisierung weist die Nadel eine Verjüngung auf, um die der Haltebereich (232) gespritzt wird, so daß ein axiales Hindurchrutschen vermieden wird. Eine Halterung der Nadel in der Hülse kann auch durch Aufrauhung der Nadel an ihrer Außenfläche, einer Verdickung oder Krümmung der Nadel im Bereich der Hülse erzielt werden. Der Antriebsstößel (210) dieser Ausführungsform weist an seinem unteren Ende eine Haltevorrichtung (211) auf, die den Haltebereich (232), wie dargestellt, formschlüssig umfaßt. Die Haltevorrichtung (211) ist seitlich offen, so daß der Stößel parallel versetzt zur Nadel mit der Haltevorrichtung (211) auf die Höhe des Haltebereiches (232) gefahren werden und durch Verschiebung quer zur Nadelachse bzw. zur Stichrichtung mit dem Haltebereich der Nadel in Eingriff gebracht werden kann. Nach diesem somit erzielten Formschluß kann die Nadel sowohl in Stichrichtung von dem Stößel (210) angetrieben werden als auch aktiv zurückgezogen werden. Im dargestellten Beispiel weist der Haltebereich der Lanzette eine Ausnehmung (Vertiefung) auf, in die bei Ankopplung ein Vorsprung der Haltevorrichtung eingreift, so daß die beiden Körper in Bezug auf die Stichrichtung weitestgehend spielfrei miteinander verbunden sind.

In der Figur 4B ist ein Magazin dargestellt, welches aus Stecheinheiten (220) gemäß Figur 4A aufgebaut ist. Bezogen auf das dargestellte Koordinatensystem kann der Antriebsstößel (210) durch Bewegung in Y-Richtung (senkrecht zur Zeichenebene) mit dem Haltebereich der Lanzette in Eingriff gebracht werden bzw. der Formschluß auch wieder aufgehoben werden. Bei aufgehobenem Formschluß kann der Antriebsstößel in X-Richtung (rechts / links) auf Höhe einer anderen Lanzette bewegt und durch Bewegung in Y-Richtung wiederum mit dieser in Eingriff gebracht werden, so daß die Lanzetten des Magazins sukzessive abgearbeitet werden können. Bei bestehendem Formschluß ist eine aktive Bewegung der Nadeln sowohl in positiver als auch negativer Z-Richtung (oben / unten) möglich.

Alternativ zu der Ankopplung gemäß Figur 4B, die sowohl eine Bewegung des Antriebsstößels in x als auch in γ-Richtung erfordert, kann auch eine Ankopplungsbewegung über eine Bewegung nur in x-Richtung erfolgen. Der Antriebsstößel weist hierzu beispielsweise zwei gegenüberliegende Haken auf, zwischen denen in x-Richtung ein Durchgang für den Haltebereich der Lanzette vorgesehen ist. Durch Bewegung in x-Richtung kann der Stößel nunmehr von Lanzette zu Lanzette bewegt werden, um einen Stichvorgang in z-Richtung auszuführen. Befindet sich der Antriebsstößel in x-Richtung auf Höhe einer Lanzette, so umfaßt die Haltevorrichtung des Stößels den Haltebereich der Lanzette formschlüssig und diese kann durch den Stößel in z-Richtung geführt bewegt werden, der Stößel führt demnach eine Stichbewegung aus und zieht auch die Lanzette wieder aktiv zurück.

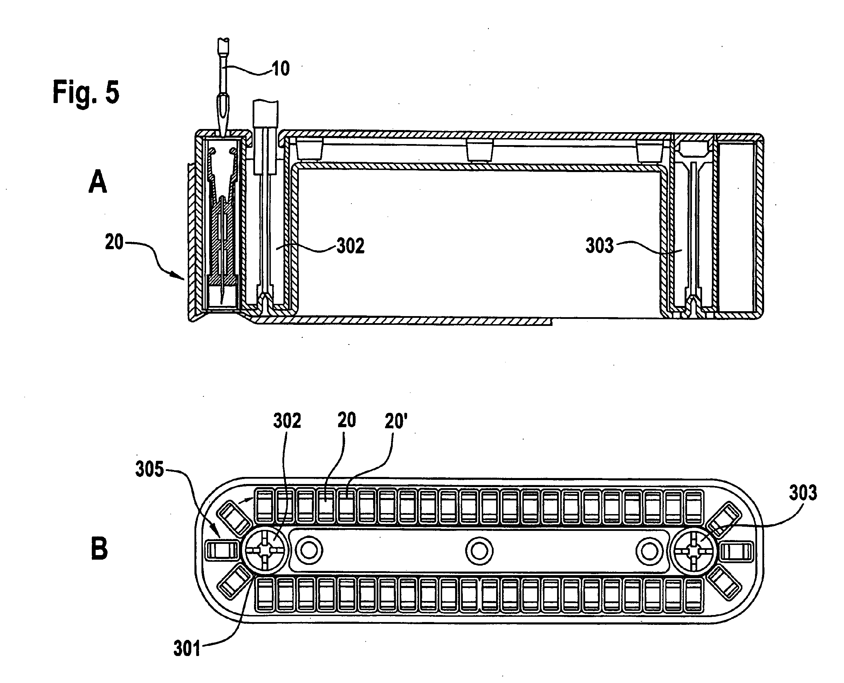

In Figur 5 ist ein automatisch arbeitendes System mit Stecheinheiten gemäß Figur 1 dargestellt. Wie aus der Aufsicht (Figur 5B) zu erkennen ist, sind die Stecheinheiten (20, 20' etc) nebeneinander an einem Transportband befestigt. Das Transportband (301) läuft um zwei voneinander beabstandete Walzen (302, 303). Eine der Walzen wird durch eine Motor angetrieben, so daß die Stecheinheiten sukzessive durch eine Ankopplungsposition (305) hindurchbewegt werden. In dieser Position ist, wie in Figur 4a dargestellt, eine formschlüssige Ankopplung eines Antriebsstößels (10) an eine in der Stechposition (305) befindliche Stecheinheit (20) möglich.

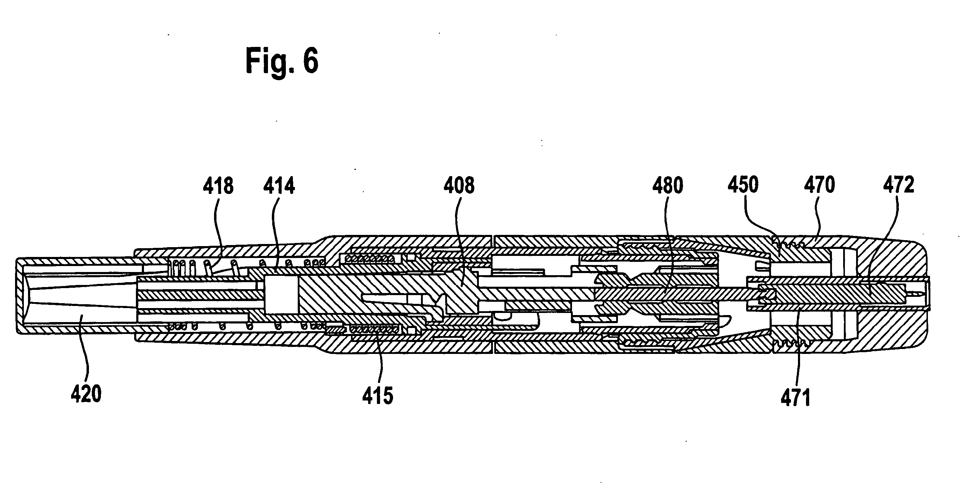

Figur 6 zeigt eine Antriebseinheit, an die eine Stecheinheit analog der Ausführungsform in Figur 1 angekoppelt ist. Das dargestellte Antriebssystem entspricht dem der europäischen Patentanmeldung Aktenzeichen 00102503.0. Bei diesem Antriebssystem wird durch das Drücken eines Druckknopfes (420) entgegen der Spannung einer Feder (418) eine Hülse (414) axial gedreht, so daß eine zweite Feder (415) gespannt wird. Die Hülse (414) wird in einer Endposition arretiert, so daß die zweite Feder (415) gespannt bleibt. Wenn der Benutzer die Arretierung aufhebt, entspannt sich die Feder (415) und die Hülse (414) wird in entgegengesetzte Richtung wie beim Spannvorgang gedreht. In der Hülse (414) befindet sich eine Nut, die als Führungskulisse für den Vortriebszylinder (408) dient, der auf seiner Außenfläche einen Zapfen oder dergleichen trägt, welcher in die Nut eingreift. Die Rotation der Hülse (414) wird so in eine Translation des Vortriebszylinders umgesetzt. Der Vortriebszylinder überträgt seinen Vortrieb auf den Antriebsstößel (480), der an seinem vorderen Ende einen Haltebereich aufweist.

Die Antriebseinheit weist weiterhin einen Haltebereich (450) auf, auf den eine Stecheinheit aufgesteckt oder aufgedreht werden kann. Die Stecheinheit beinhaltet eine Kappe (470), die eine Fläche zum Andrücken an die Hautoberfläche besitzt. In der Kappe ist eine Hülse (471) angeordnet, in welcher eine Lanzette (472) mit Haltevorrichtungen an ihrer, der Nadelspitze abgewandten Seite, aufweist. Die Haltevorrichtung der Lanzette entspricht den Haltevorrichtungen (32a, 32b) der Figur 1. Aus Figur 5 ist weiterhin zu erkennen, daß eine formschlüssige Verbindung von Lanzette und Antriebsstößel bereits durch Anbringen der Kappe (470) an der Antriebsvorrichtung erzielt wird.

Figur 7 zeigt ein System zur Entnahme von Körperflüssigkeiten, das eine Vielzahl von Ähnlichkeiten zu dem in Figur 3 dargestellten System aufweist. Insbesondere wird auf die bei Figur 3 und Figur 6 erfolgte Beschreibung des Antriebes und Spannmechanismus verwiesen. Das System gemäß Figur 7 weist eine Stecheinheit (120') mit einer Kappe (121') und einer Lanzette (130') auf. In der Kappe (121') befindet sich ein axialer Durchgang, durch den der Lanzettenkörper beim Stechvorgang hindurchtreten kann. Vorzugsweise sind sowohl Durchgang als auch Lanzettenkörper so aufeinander abgestimmt, daß eine axiale Führung der Lanzette beim Stechvorgang mit lediglich geringem Spiel in Querrichtung gegeben ist. An ihrem hinteren Ende weist die Kappe ein Gewinde (126) auf, das auf ein entsprechendes Gewinde (127) der Antriebseinheit (100') aufgeschraubt werden kann. An dem der Nadelspitze entgegengesetzten Ende besitzt die Lanzette einen oder auch mehrere (im dargestellten Fall 2) Zapfen (131'), die beim Aufsetzen bzw. Aufdrehen der Kappe auf die Antriebseinheit in Formschluß mit der Haltevorrichtung (132') gelangen. Die Haltevorrichtung weist hierzu eine Ausnehmung oder Nut auf, die einen axialen Teil (134a) sowie einen quer dazu angeordneten Teil (134b) aufweist. Beim Aufsetzen der Kappe auf die Antriebseinheit gelangen die Zapfen (131') in den axialen Teil der Nut (134) und durchfahren diesen bis auf Höhe des quer angeordneten Teiles der Nut. Durch Aufdrehen der Kappe (120') auf die Antriebseinheit (100') wird der Zapfen (131') vom Ende des axialen Teils in dem Querteil der Nut bis zum gegenüberliegenden Ende verfahren. Wie aus Figur 7II zu erkennen ist, wird die Lanzette mittels der Zapfen durch die Haltevorrichtung (132') axial gehaltert, so daß eine geführte Stechbewegung mit der Lanzette erfolgen kann. Durch die Lagerung der Zapfen im Querteil der Nuten kann mit der Lanzette sowohl eine Bewegung zum Austreten der Nadelspitze als auch ein Zurückziehen erfolgen. Wie aus Figur 7 ersichtlich ist, kann die formschlüssige Verbindung zwischen Lanzette und Haltevorrichtung erzielt werden, ohne daß ein Verklemmen oder Verrasten erfolgt. Das in Figur 7 dargestellte Kopplungsprinzip von Lanzette und Antriebsstößel ist selbstverständlich auch reziprok, d. h. mit einer entsprechenden Haltevorrichtung an der Lanzette und einem Haltebereich an dem Stößel bzw. Antrieb möglich.

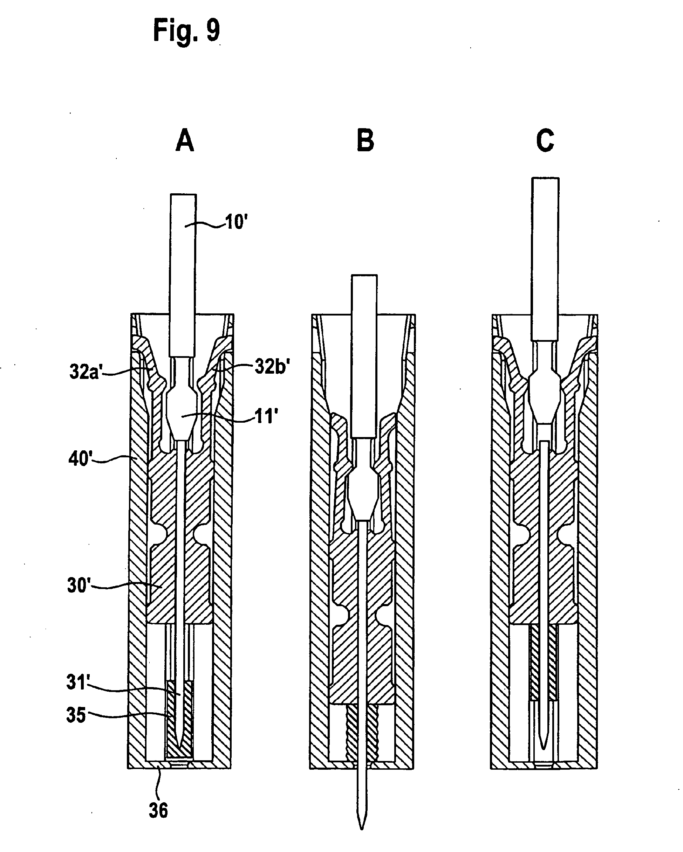

Figur 9 zeigt eine Weiterentwicklung des Systems aus Figur 1. Der Antriebsstößel (10') weist einen Haltebereich (11') auf, der an seiner Oberseite eine umlaufende, schräge Fläche besitzt, die, wie in Figur 9B zu erkennen, beim Stechvorgang in Passung mit schrägen Flächen der Haken (32a', 32b') kommt. Das Ende des Antriebsstößels sitzt auf dem Nadelende auf, während das Zusammendrücken der in sich flexiblen Haken (32a', 32b') bewirkt, daß Schrägen an der Hakeninnenseite an Schrägen an der Oberseite des Haltebereiches gedrückt werden, so daß der Haltebereich auf das Nadelende gedrückt wird und eine in Stichrichtung spielfreie Verbindung von Haltebereich und Lanzette entsteht. Durch diese Passung kann eine sehr präzise formschlüssige Halterung zwischen Lanzette und Antriebsstößel erreicht werden, die (Herstellungs-)Toleranzen ausgleicht, so daß sowohl beim Stich als auch bei der Rückziehbewegung ein Spiel vermieden wird.
Die Haltevorrichtung in Form der Haken (32a', 32b') ist ferner so gestaltet, daß die freien Enden der Haken in Ausnehmungen in der Hülse (40') eingreifen. Hierdurch wird vermieden, daß die Lanzette (30') aus der Hülse (40') ungewollt herausrutschen kann. Wie aus dem Übergang von Figur 9A zu Figur 9B zu erkennen ist, werden die freien Enden der Haken beim Beginn des Stechvorganges zunächst aus den Ausnehmungen gelöst und die Haken werden beim Einschieben in die sich verjüngende Hülse zusammengedrückt, so daß sie den Haltebereich des Antriebsstößels umschließen.

In der Figur 9 ist weiterhin zu erkennen, daß die Nadelspitze (31') in einem Material (35) angeordnet ist. Dieses Material (35) ist vorzugsweise ein Elastomer, das die Nadelspitze dicht umgibt, so daß eine Kontamination der Nadelspitze wirksam verändert wird. Als geeignete Elastomere kommen in Frage: Styrol-Oligoblock-Copolymere, thermoplastische Polyolefine, thermoplastische Polyurethane, thermoplastische Copolyester und thermoplastische Copolyamide. Bezüglich weiterer Details zu dieser Vorgehensweise, eine Kontamination der Lanzettenspitze zu vermeiden, wird hiermit auf die internationale Anmeldung WO 01/66010 Bezug genommen. In der Ausgangsposition gemäß Figur A vor dem Stich, befindet sich die Nadelspitze in dem Elastomer und wird beim Durchführen eines Stiches von der Nadel durchstochen, wie aus Figur B zu erkennen. Hierzu weist die Hülse (40') an ihrer Unterseite eine Platte (36) mit zentraler Öffnung auf. Die Platte verhindert ein Austreten des Elastomers durch die Öffnung, so daß dieses durchstochen wird, während die Nadel durch die zentrale Öffnung austritt. Beim Zurückziehen der Lanzette verbleibt das Elastomer an der Nadel, wobei jetzt die Nadelspitze frei liegt (siehe Figur C).

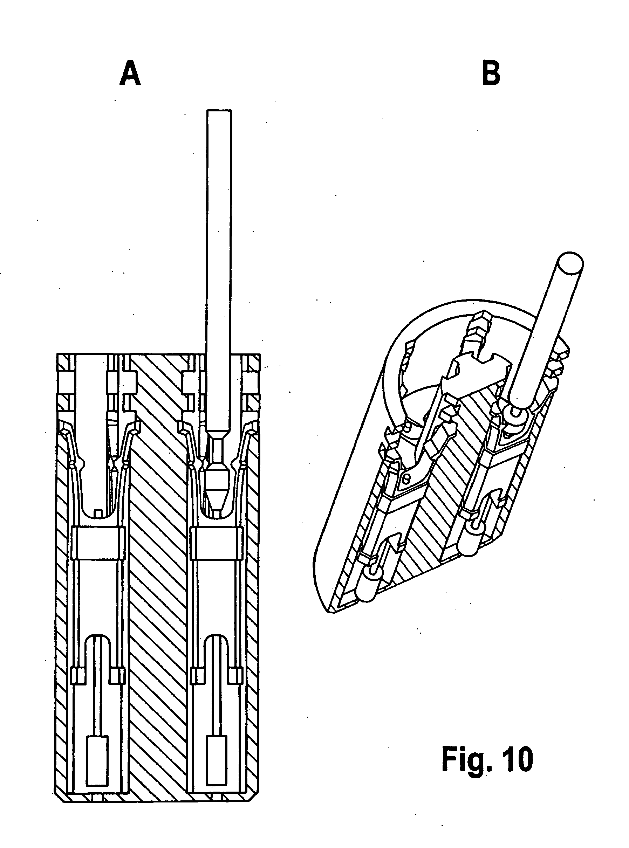

Figur 10 zeigt im Querschnitt (Figur A) und in perspektivischer Ansicht (Figur B) ein zylindrisches Magazin auf Basis von Lanzetten gemäß Figur 1 bzw. Figur 9. Mit einem solchen Magazin ist es möglich, auf einfache Weise neue Lanzetten an den Antrieb anzukoppeln. Hierzu kann beispielsweise der Antriebsstößel ortsfest bezüglich einer Stechhilfe angeordnet sein und das in Figur 10 darstellte, trommelförmige Magazin wird ähnlich einer Revolvertrommel gedreht, so daß ungebrauchte Lanzetten in die Position zur Ankopplung des Antriebsstößels gelangen.

## Patentansprüche

1. Verfahren zum zeitweisen Ausführen einer Nadel aus einer Vorrichtung mit den Schritten
- Formschlüssige Ankopplung einer Lanzette (30, 130, 130') an einen Stößel (10, 110) einer Antriebseinheit, wobei entweder der Stößel einen Haltebereich (11) und die Lanzette eine Haltevorrichtung (32a, 32b) oder die Lanzette einen Haltebereich (131) und der Stößel eine Haltevorrichtung (132a, 132b) aufweist und die Haltevorrichtung der Lanzette oder des Stößels mindestens ein bewegliches Element (32a, 32b/132a, 132b) aufweist, **dadurch gekennzeichnet, dass** bevor der Stößel an die Lanzette ankoppelt, das mindestens eine bewegliche Element sich in einer offenen Position befindet und während einer Längsbewegung der Haltevorrichtung sich das mindestens eine bewegliche Element quer zur Einstichrichtung bewegt, sodaß das mindestens eine bewegliche Element sich um den Haltebereich schließt, wobei eine formschlüssige Kopplung von Stößel und Lanzette erfolg und
- Bewegen des Stößels aus einer Ruheposition bei der die Nadel der Lanzette innerhalb einer Stecheinheit angeordnet ist in eine Position, in der die Nadel aus einer Austrittsöffnung der Stecheinheit austritt,
- Zurückbewegen des Stößels um die Nadel in die Stecheinheit zurückzuziehen

2. Verfahren gemäß Anspruch 1, bei dem die Ankopplung der Lanzette an die Antriebseinheit durch Anbringen der Stecheinheit an der Antriebseinheit erfolgt

3. Verfahren gemäß Anspruch 1, bei dem die Kopplung von Lanzette und Stößel durch Abnehmen der Stecheinheit von der Antriebseinheit gelöst wird.

## Claims

1. Method for temporarily extending a needle from a device comprising the steps
- coupling a lancet (30, 130, 130') to a plunger (10, 110) of a drive unit in a form-fitting manner, where either the plunger has a holding area (11) and the lancet has a holding device (32a, 32b) or the lancet has a holding area (131) and the plunger has a holding device (132a, 132b) and the holding device of the lancet or of the plunger has at least one movable element (32a, 32b/132a, 132b), **characterized in that** before the plunger couples to the lancet the at least one movable element is in an open position and during a longitudinal movement of the holding device the at least one movable element moves transversely to the lancing direction such that the at least one movable element closes around the holding area to make a form-fitting coupling of the plunger and the lancet and
- moving the plunger from a resting position in which the needle of the lancet is located inside a lancing unit into a position in which the needle emerges from an exit opening of the lancing unit,
- moving the plunger back in order to retract the needle into the lancing unit.

2. Method according to claim 1, in which the lancet is coupled to the drive unit by attaching the lancing unit to the drive unit.

3. Method according to claim 1, in which the lancet and plunger are disconnected by removing the lancing unit from the drive unit.

## Revendications

1. Procédé pour mettre en oeuvre temporairement une aiguille à partir d'un dispositif présentant les étapes suivantes
- rattachement par complémentarité de formes d'une lancette (30, 130, 130') à un coulisseau (10, 110) d'une unité d'entraînement, sachant que soit le coulisseau présente une zone de retenue (11) et la lancette un dispositif de retenue (32a, 32b) ou la lancette présente une zone de retenue (131) et le coulisseau un dispositif de retenue (132a, 132b) et le dispositif de retenue de la lancette ou du coulisseau présente au moins un élément mobile (32a, 32b/132a, 132b), **caractérisé en ce que**, avant que le coulisseau soit rattaché à la lancette, le au moins un élément mobile se trouve dans une position ouverte et le au moins un élément mobile se déplace transversalement au sens de piquage pendant un déplacement longitudinal du dispositif de retenue, de sorte que le au moins un élément mobile se ferme autour de la zone de retenue, un accouplement par complémentarité de formes du coulisseau et de la lancette s'effectuant et
- déplacement du coulisseau à partir d'une position de repos, dans laquelle l'aiguille de la lancette est disposée à l'intérieur d'une unité de piquage, dans une position dans laquelle l'aiguille sort d'une position de sortie de l'unité de piquage
- déplacement en arrière du coulisseau pour retirer l'aiguille dans l'unité de piquage.

2. Procédé selon la revendication 1, dans lequel le rattachement de la lancette à l'unité d'entraînement s'effectue par la mise en place de l'unité de piquage sur l'unité d'entraînement.

3. Procédé selon la revendication 1, dans lequel l'accouplement de la lancette et du coulisseau est détaché par l'enlèvement de l'unité de piquage de l'unité d'entraînement.
